# FASCICULE DE BREVET EUROPEEN

(11) **EP 2 303 208 B1**
(45) Date de publication et mention de la délivrance du brevet: **04.09.2019**
(21) Numéro de dépôt: 09784502.8
(22) Date de dépôt: 09.07.2009
(51) Int. Cl.: A61F 13/02

(54) **PROCEDE DE FABRICATION DE PANSEMENTS CONTENANT AU MOINS UN ACTIF**
VERFAHREN ZUR HERSTELLUNG VON VERBÄNDEN MIT MINDESTENS EINEM WIRKSTOFF
METHOD OF MANUFACTURING DRESSINGS CONTAINING AT LEAST ONE ACTIVE INGREDIENT

(30) Priorité: 10.07.2008 FR 0854685
(43) Date de publication de la demande: 06.04.2011
(73) Titulaire: Urgo Recherche Innovation et Développement, 21300 Chenove (FR)
(72) Inventeur: FREROT, Denis, F-21910 SAULON LA RUE (FR)
(74) Mandataire: Hubert, Philippe
(86) Numéro de dépôt international: PCT/FR2009/051373
(87) Numéro de publication internationale: WO 2010/004222

(56) Documents cités:
- WO-A-96/10398
- DE-A1- 1 461 280
- FR-A- 2 798 856
- US-A- 5 505 958
- US-A- 5 773 022

## Description

La présente invention concerne généralement un procédé de fabrication de pansements, en particulier de pansements présentant des bords amincis, contenant au moins un actif, ledit actif étant plus particulièrement incorporé au niveau de la surface destinée à venir en contact avec la plaie et/ou la peau.

Des pansements à bords amincis sont généralement constitués d'un matériau adhérent malléable comportant, sur l'une de ses faces destinée à venir au contact de la plaie et/ou de la peau, un film de protection formant une ailette détachable lors de l'utilisation, et sur la face opposée un film formant support.

Des procédés de fabrication de pansements à bords amincis sont connus et notamment décrits dans les brevets EP 0 573 708, EP 0 756 854, EP 0 818 187 et EP 0 999 025, FR2798856, US5505958, WO9610398.

Ainsi, le brevet EP 0 573 708 décrit un procédé de fabrication en continu de pansements comprenant :
- une première étape au cours de laquelle un film de protection et un matériau adhérent malléable sont solidarisés entre eux et mis en forme au niveau d'un poste de moulage ;
- une deuxième étape au cours de laquelle un film support est complexé au matériau adhérent au niveau de sa face opposée à la face portant le film de protection ; et
- une troisième étape au cours de laquelle l'ensemble ainsi constitué est découpé en pansements individuels.

Le brevet EP 0 756 854 décrit un procédé de fabrication de pansements en continu dans lequel les différentes couches constitutives (film support, matériau adhérent malléable et film de protection) sont solidarisées et mis en forme simultanément au niveau d'un poste de laminage-moulage alimenté :
- d'une part, par un élément stratifié préalablement constitué en déposant le matériau adhérent malléable constitué d'une masse contenant des hydrocolloïdes sur un film support ; et
- d'autre part, par un film de protection.

Ce procédé comporte une seconde étape au cours de laquelle l'ensemble préalablement mis en forme est découpé en pansements individuels.

Le brevet EP 0 818 187 décrit un procédé de fabrication de pansements dans lequel les différentes couches constitutives sont mises en forme et découpées en pansements individuels au cours de la même étape à l'aide d'un poste de travail unique.

Il est à noter que selon ce document, le film support est constitué par un film bicouche dont l'une des couches est pelable et destinée à protéger le matériau adhérent malléable lors de sa mise en forme et de sa découpe simultanée.

Le brevet EP 0 999 025 décrit un procédé de fabrication de pansements analogue au procédé décrit dans le document EP 0 756 854 dans lequel un film support bicouche comprenant une couche délaminable est utilisé lors de la première étape de mise en forme du pansement et retiré préalablement à la seconde étape de découpe en pansements individuels.

Aucun des procédés décrits dans ces brevets antérieurs n'envisage l'incorporation d'un ou plusieurs actifs au niveau de la surface du matériau adhérent malléable destinée à venir en contact avec la plaie et/ou la peau.

En effet, dans chacun des procédés décrits dans ces brevets, le film de protection destiné à former l'ailette détachable du pansement avant utilisation est solidarisé au matériau adhérent malléable préalablement à ou au cours de l'étape de moulage.

La mise en oeuvre de ces procédés connus pour la fabrication d'un pansement contenant au moins un actif nécessiterait donc l'incorporation préalable de l'actif au cours de la fabrication du matériau malléable adhérent.

Or, la fabrication du matériau malléable adhérent est généralement réalisée à une température élevée, de sorte que l'incorporation d'actifs au sein de ce matériau serait limitée aux seuls actifs insensibles à la température.

De plus, en incorporant l'actif au cours de cette étape, celui-ci se trouverait disséminé dans l'ensemble de la masse constituant le matériau malléable adhérent et ne serait donc pas directement disponible au niveau de la plaie.

Dans ces conditions, la présente invention a pour but de résoudre le problème technique consistant en la fourniture d'un nouveau procédé de fabrication de pansements, de préférence à bords amincis, permettant l'incorporation d'un ou plusieurs actifs au niveau de la surface destinée à venir en contact avec la plaie et/ou la peau, sans affecter les propriétés d'adhérence de celle-ci.

La présente invention a également pour but de résoudre le problème technique précité de façon simple et utilisable à l'échelle industrielle.

Ainsi, la présente demande vise à couvrir un procédé de fabrication de pansements, selon la revendication 1, de préférence à bords amincis, caractérisé en ce qu'il comprend les étapes consistant :
a) à former un premier élément stratifié en solidarisant entre eux un film support et une couche d'un matériau malléable adhérent ;
b) à mettre en forme ledit élément stratifié pour obtenir un produit intermédiaire comportant une pluralité de zones formant chacune une ébauche de pansement ;
c) à traiter le produit intermédiaire ainsi obtenu pour déposer un ou plusieurs actifs sur la surface libre dudit matériau malléable adhérent ;
d) à former un second élément stratifié en solidarisant entre eux ledit produit intermédiaire ainsi traité et un film de protection ;
e) à découper ledit second élément stratifié pour former ainsi une pluralité de pansements.

Ce procédé présente de nombreux avantages.

Tout d'abord, le pansement obtenu par la mise en oeuvre de ce procédé présente une disponibilité améliorée de l'actif, du fait que celui-ci est directement présent au niveau de la surface destinée à venir en contact avec la plaie et/ou la peau. En outre, l'actif peut être disposé aisément, de façon localisée sur la surface du pansement destinée à couvrir la zone à traiter.

De ce fait, la quantité d'actifs par pansement peut être réduite en limitant ainsi le coût de fabrication.

Le film support utilisé dans le cadre de la présente invention peut être constitué de matériaux polymériques de nature variée tels qu'un polyamide, polyuréthane, polyester, polyéther, polychlorure de vinyle, polychlorure de vinylidène, alcool polyvinylique, polyacétate de vinyle, polystyrène, fluorure polyvinylique, une polyoléfine comme par exemple un polyéthylène ou un polypropylène, un matériau à base de copolymère de polyéther polyester, de copolymère de polyester ou polyéther polyuréthane, de copolymère polyéther polyamide.

Ce film support est en général un film mince dont l'épaisseur peut être comprise entre 5 et 150 µm, de préférence entre 15 et 70 µm.

Ce film support peut être adhésivé ou non sur la face destinée à venir en contact avec le matériau malléable adhérent.

Ce film support peut être monocouche ou multicouches, comme par exemple un film bicouche dont la seconde couche forme la surface opposée à celle destinée à venir en contact avec le matériau malléable adhérent. Cette seconde couche peut être constituée d'un matériau polymérique de même nature que ceux mentionnés précédemment ou bien encore de papiers siliconés ou non.

Avantageusement, le film support est composé d'un film bicouche constitué d'une couche de polyuréthane non adhésivée destinée à venir en contact avec le matériau malléable adhérent complexée à une couche de polyester ou de papier.

Le film support peut également consister en une mousse ayant une épaisseur de 10 à 500 µm.

Dans le cadre de la présente description, par "matériau malléable", on entend désigner tout matériau qui sous l'action de la température (par exemple par chauffage) perd son élasticité.

Un tel matériau peut être de nature variée et constitué des matières habituellement utilisées à cet effet dans les pansements.

D'une façon générale, ce matériau est constitué d'une matrice élastomérique comprenant des hydrocolloïdes.

Par "matrice élastomérique", on entend désigner ici les compositions comprenant :
- un ou plusieurs élastomères choisis de préférence parmi les copolymères séquencés poly(styrène-oléfine-styrène) ;
- un ou plusieurs composés parmi les produits dits "tackifiants", les antioxydants, les plastifiants liquides et les agents permettant d'améliorer la cohésion tels que les caoutchoucs butyle ou des polyisobutylènes de haut poids moléculaire.

De telles compositions sont ainsi définies dans "Advances in Pressure Sensitive Adhesive Technology" édité par Donatas Satas en avril 1995 dans le chapitre 7 "Wound dressings" pages 158 à 171 et sont aussi parfaitement connues de l'homme de l'art.

Avantageusement, dans le cadre de la présente invention, on utilisera un matériau malléable adhérent constitué d'une matrice élastomérique incorporant des quantités plus ou moins importantes d'hydrocolloïdes, désignés par la suite par l'expression "masses hydrocolloïdes".

Par "hydrocolloïde", on entend désigner ici les composés utilisés par l'homme de l'art pour leur aptitude à absorber les liquides hydrophiles tels que l'eau, le sérum physiologique ou les exsudats d'une plaie.

Comme hydrocolloïdes appropriés, on peut citer par exemple la pectine, les alginates, les gommes végétales naturelles telle la gomme de Karaya, les dérivés de cellulose telles les carboxyméthylcelluloses et leurs sels de métal alcalin tels le sodium ou le calcium ainsi que les polymères synthétiques à base de sels de l'acide acrylique, connus sous l'appellation "superabsorbants", comme par exemple les produits commercialisés par la société BASF sous la dénomination LUQUASORB® 1003 et par la société CIBA Specialty chemicals sous la dénomination SALCARE® SC91. Des mélanges de ces produits peuvent être utilisés à titre d'hydrocolloïde.

Dans le cadre de la présente invention par "actif", on entend désigner tout composé ou composition présentant des propriétés pharmacologiques dermatologiques ou cosmétiques utiles dans le traitement de la peau ou dans le traitement des plaies.

A titre d'exemple, on peut citer les agents anti-inflammatoires, analgésiques, antiseptiques ou antibactériens, les agents favorisant la cicatrisation ou permettant de nettoyer la plaie, les agents anesthésiques topiques, les agents kératolytiques. Un ou plusieurs agents appartenant aux classes précitées peuvent être utilisés à titre d'actifs.

Ces actifs pourront se présenter sous différentes formes et en particulier sous forme de poudre ou de liquide (solution ou dispersion).

Ces actifs peuvent en outre être conditionnés sous forme de microcapsules. Ces microcapsules seront de préférence constituées d'un matériau hydrosoluble apte à se dissoudre dans les exsudats de la plaie pour libérer les actifs au niveau de la plaie. La cinétique de relargage de l'actif ou des actifs peut être régulée par un choix judicieux du matériau constituant la capsule.

La granulométrie des poudres ou microgranules d'actif(s) peut varier dans une large mesure. De façon générale, cette granulométrie peut être de l'ordre de 200 à 400 micromètres.

La granulométrie des microcapsules incorporant l'actif ou les actifs peut être comprise entre 1 µm et 1 mm, de préférence entre 150 et 600 µm, de préférence encore de l'ordre de 400 µm.

Lorsque plusieurs actifs sont incorporés aux pansements selon l'invention, ceux-ci peuvent être utilisés sous une forme unique, comme par exemple un mélange de poudres ou une solution ou dispersion, ou bien encore sous des formes distinctes comme par exemple une poudre et un liquide.

La quantité d'actif(s) incorporé(s) dépendra de la nature de l'actif et pourra être facilement déterminée par l'homme du métier. D'une façon générale, la quantité d'actif(s) incorporé(s) est de l'ordre de 0,01 à 0,25 g d'actif(s) par centimètre carré de surface active de pansement c'est-à-dire de surface destinée à venir en contact avec la plaie et/ou la peau.

Le film de protection utilisé dans le cadre de la présente invention est destiné à constituer une ailette de protection du matériau malléable adhérent (masse hydrocolloïde) destinée à être retirée préalablement à l'utilisation du pansement sur la plaie et/ou la peau.

Ce film de protection peut être constitué de matériaux variés tels que les matériaux habituellement utilisés par l'homme du métier pour protéger la surface adhésive d'un pansement. Il peut s'agir en particulier d'un papier, d'un polyester, d'un polypropylène ou d'un carton siliconé.

L'invention sera mieux comprise et d'autres caractéristiques de celle-ci apparaîtront plus clairement à la lecture de la description explicative qui va suivre faite en référence aux dessins schématiques annexés dans lesquels :
- la figure 1 illustre de façon schématique un premier mode de réalisation du procédé selon l'invention dans lequel les pansements sont fabriqués de façon continue ;
- la figure 2 illustre de façon schématique un second mode de réalisation du procédé selon l'invention dans lequel les pansements sont fabriqués de façon discontinue ou séquentielle.

Dans ces figures, les éléments identiques portent les mêmes chiffres de référence.

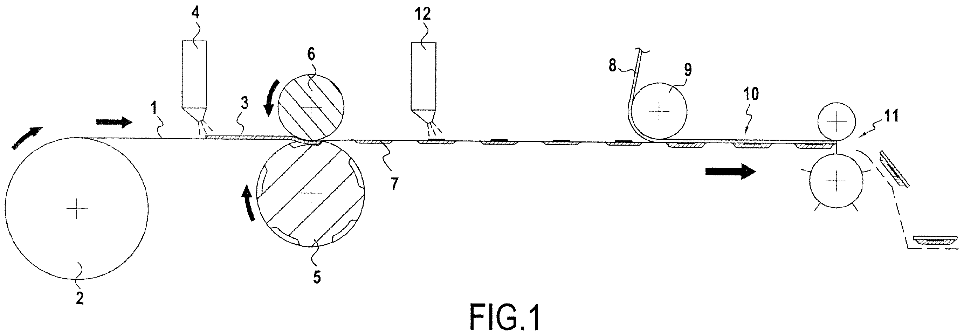

En référence à la figure 1, on décrira tout d'abord un premier mode de réalisation du procédé selon l'invention dans lequel les pansements sont fabriqués de façon continue.

Un ensemble constitué d'un film support 1 et d'un matériau malléable adhérent 3 est apporté de façon continue au niveau d'un poste de "laminage-moulage" constitué :
- d'un premier cylindre 5 comportant un ensemble d'empreintes présentant chacune la forme générale du pansement à obtenir, de préférence un pansement présentant des bords amincis ; et
- d'un second cylindre 6 dont la surface externe est sensiblement plane et lisse et constituée d'un matériau anti-adhérent.

Dans l'exemple représenté, l'ensemble "film support-matériau malléable" alimentant le poste de laminage-moulage est réalisé en déposant par enduction, au moyen de la tête 4 d'une buse, un matériau malléable 3 constitué d'une matrice élastomérique comprenant des hydrocolloïdes préalablement chauffée à une température de 100 à 160°C, sur un film support alimenté de façon continue par une bobine d'alimentation 2.

Lors du passage de l'ensemble ainsi constitué dans le poste de laminage-moulage, la température du matériau malléable adhérent est de l'ordre de 80 à 120°C.

Selon une variante de réalisation, le matériau malléable adhérent peut être extrudé et apporté directement au niveau des cylindres 5 et 6. Dans ce cas, la température de ce matériau est de l'ordre de 70 à 90°C.

Lors du passage dans le poste de laminage-moulage, le matériau malléable 3 adhérent et le film support 1 sont simultanément solidarisés entre eux et mis en forme.

Le cylindre 6 présente avantageusement une surface lisse constituée d'un matériau anti-adhérent choisi pour éviter le matériau malléable adhérent 3 n'adhère audit cylindre. A cet effet, le cylindre 6 peut être recouvert d'une substance anti-adhérente à base de silicone.

Alternativement, le cylindre 6 peut présenter des formes convexes ou des empreintes, au niveau de sa surface afin de former une légère dépression au niveau de la surface du pansement destinée à recevoir les actifs et faciliter ainsi leur incorporation.

A la sortie du poste de laminage-moulage, on obtient un produit intermédiaire 7 comportant une pluralité de zones présentant la forme générale des empreintes présentes sur le cylindre 5 et formant chacune une ébauche de pansement, de préférence à bords amincis.

Le produit intermédiaire 7 ainsi obtenu est traité pour déposer un ou plusieurs actifs sur la surface libre des portions comprenant le matériau malléable adhérent 3.

Cette opération est avantageusement réalisée par saupoudrage au moyen d'un distributeur de poudre 12 connu de l'homme du métier, lorsque l'actif (les actifs) se présente(nt) sous forme de poudre ou de microcapsule.

Selon une variante de réalisation, cette opération peut être réalisée au moyen d'un pistolet ou d'une buse lorsque l'actif (les actifs) se présente(nt) sous forme liquide ou semi-liquide.

Avantageusement, l'actif (les actifs) est(sont) déposé(s) au niveau des parties du matériau malléable destinées à constituer les parties centrales des pansements.

Selon une variante de réalisation, l'actif (les actifs) est(sont) déposé(s) de façon à former des motifs à la surface du pansement comme par exemple un carré, un rectangle ou une ou plusieurs lignes.

Selon une autre variante de réalisation, l'actif (les actifs) est(sont) déposé(s) sous forme de pastilles disposées au niveau des zones destinées à constituer les parties centrales des pansements ou bien encore réparties de façon régulière ou non à la surface des zones destinées à constituer les pansements. Ces pastilles peuvent avoir un diamètre de l'ordre de 6 à 8 millimètres pour un pansement présentant une surface totale de l'ordre de 1 à 3 cm², de préférence 1,5 à 2,5 cm².

Le produit intermédiaire 7 ainsi traité est ensuite solidarisé à un film de protection 8 alimentant de façon continue un cylindre de laminage 9. Ce film de protection est destiné à constituer des ailettes de protection détachables des pansements avant utilisation.

On obtient ainsi un second élément stratifié 10.

Ce second élément alimente de façon continue un poste de découpe 11 connu en soi pour former une pluralité de pansements individuels.

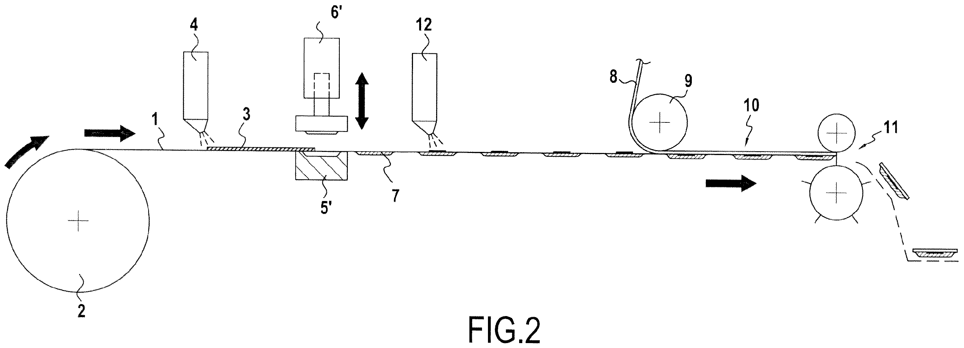

On a représenté à la figure 2 un second mode de réalisation du procédé conforme à l'invention dans lequel les pansements sont fabriqués de façon discontinue ou séquentielle.

A la différence du mode de réalisation représenté à la figure 1, le poste de laminage-moulage comprend ici deux plaques déplaçables relativement l'une vers l'autre, la première plaque (5') comprenant un ensemble d'empreintes présentant chacune la forme générale du pansement à obtenir et une seconde plaque (6') dont la surface est constituée d'un matériau anti-adhérent. Dans l'exemple représenté, la première plaque (5') est fixe, seule la seconde plaque (6') est déplaçable longitudinalement selon un axe sensiblement perpendiculaire au plan formé par l'ensemble constitué du film (1) et du matériau adhérent malléable (3). Bien entendu, d'autres configurations sont possibles, par exemple une configuration dans laquelle les deux plaques (5', 6') sont longitudinalement déplaçables.

Pour le reste, ce second mode de réalisation est analogue au premier mode de réalisation décrit précédemment et permet d'obtenir les mêmes pansements.

## Revendications

1. Procédé de fabrication de pansements, de préférence à bords amincis, **caractérisé en ce qu'**il comprend les étapes consistant :
a) à former un premier élément stratifié en solidarisant entre eux un film support (1) et
une couche d'un matériau malléable adhérent destinée à venir en contact avec la plaie et contenant:
- un ou plusieurs élastomères choisis parmi les copolymères séquencés poly(styrène-oléfine-styrène),
- un ou plusieurs composés choisis parmi les produits dits "tackifiants", les antioxydants, les plastifiants liquides, et les agents permettant d'améliorer la cohésion choisis parmi les caoutchoucs butyles et les polyisobutylènes de haut poids moléculaire,
- des hydrocolloïdes;
b) à mettre en forme ledit élément stratifié pour obtenir un produit intermédiaire (7) comportant une pluralité de zones formant chacune une ébauche de pansement ;
les étapes a) et b) précitées étant réalisées simultanément au niveau d'un poste de laminage-moulage ;
c) à traiter le produit intermédiaire (7) ainsi obtenu pour déposer un ou plusieurs actifs sur la surface libre dudit matériau malléable adhérent ;
d) à former un second élément stratifié (10) en solidarisant entre eux ledit produit intermédiaire (7) ainsi traité et un film de protection (8) ;
e) à découper ledit second élément stratifié (10) pour former ainsi une pluralité de pansements.

2. Procédé de fabrication selon la revendication 1, **caractérisé en ce que** le poste de laminage-moulage précité comprend un moule (5, 5') destiné à être mis en contact avec le film support (1) et un contre-moule (6, 6') comportant une face constituée d'un matériau anti-adhérent destiné à venir en contact avec le matériau malléable adhérent (3).

3. Procédé selon la revendication 2, **caractérisé en ce que** le poste de laminage-moulage précité est alimenté de façon continue et comprend un premier cylindre (5) présentant une pluralité d'empreintes ayant chacune la forme générale du pansement à réaliser et un second cylindre (6) dont la surface externe est sensiblement plane et lisse et constituée d'un matériau anti-adhérent.

4. Procédé de fabrication selon la revendication 2, **caractérisé en ce que** le poste de laminage-moulage précité est alimenté de façon discontinue et comprend deux plaques déplaçables l'une vers l'autre, la première plaque (5') comprenant un ensemble d'empreintes présentant chacune la forme générale du pansement à obtenir et une seconde plaque (6') dont la surface est constituée d'un matériau anti-adhérent.

5. Procédé selon l'une quelconque des revendications précédentes, **caractérisé en ce qu'**il comprend une étape préalable à l'étape a) précitée consistant à alimenter le poste de laminage-moulage par un ensemble formé d'un film support (1) portant sur l'une de ses faces une couche d'un matériau malléable adhérent (3), ledit ensemble étant obtenu par enduction, extrusion ou par dépose.

6. Procédé selon l'une quelconque des revendications précédentes, **caractérisé en ce que** ledit(s) actif(s) est(sont) déposé(s) sous forme solide, par exemple sous forme de poudre, ou sous forme liquide, par exemple sous forme de solution ou de suspension ou bien encore sous forme semi-liquide comme par exemple sous forme de pâte ou de gel.

7. Procédé selon la revendication 6, **caractérisé en ce que** ledit(s) actif(s) est(sont) déposé(s) sous forme de poudre au moyen d'un distributeur de poudres (12), de préférence au niveau des parties du matériau malléable adhérent destinées à constituer les parties centrales des pansements.

8. Procédé selon la revendication 7, **caractérisé en ce que** ledit(s) actif(s) est(sont) déposé(s) sous forme de pâte ou de gel, au moyen d'un pistolet ou d'une buse, de préférence au niveau des parties du matériau malléable adhérent destinées à constituer les parties centrales des pansements.

## Patentansprüche

1. Verfahren zur Herstellung von Verbänden, vorzugsweise mit schlankeren Kanten, **dadurch gekennzeichnet, dass** es die Schritte umfasst, die darin bestehen:
a) ein erstes Schichtelement durch festes Verbinden einer Trägerfolie (1) und einer Schicht aus einem formbaren Klebematerial auszubilden, die dazu bestimmt ist, mit der Wunde in Kontakt zu kommen, und umfasst:
- ein oder mehrere Elastomere, ausgewählt aus den Poly(styrol-Olefin-Styrol)-Blockcopolymeren,
- eine oder mehrere Verbindungen, ausgewählt aus den sogenannten "Tackifiern", den Antioxidantien, den flüssigen Weichmachern und den Mitteln, die ermöglichen, die Kohäsion zu verbessern, ausgewählt aus den Butylkautschuken und den hochmolekularen Polyisobutylenen,
- Hydrokolloide,
b) das Schichtelement in Form zu bringen, um ein Zwischenprodukt (7) mit einer Vielzahl von Bereichen zu erhalten, die jeweils einen Verbandrohling bilden,
wobei die vorgenannten Schritte a) und b) im Bereich einer Walz-Form-Station gleichzeitig durchgeführt werden,
c) das so erhaltene Zwischenprodukt (7) zu behandeln, um einen oder mehrere Wirkstoffe auf der freien Oberfläche des formbaren Klebematerials abzuscheiden,
d) ein zweites Schichtelement (10) zu bilden, indem das so behandelte Zwischenprodukt (7) und eine Schutzfolie (8) fest untereinander verbunden werden,
e) das zweite Schichtelement (10) zu zerschneiden, um dadurch eine Vielzahl von Verbänden zu bilden.

2. Herstellungsverfahren nach Anspruch 1, **dadurch gekennzeichnet, dass** die vorgenannte Walz-Form-Station eine Form (5, 5'), welche dazu bestimmt ist, mit der Trägerfolie (1) in Kontakt gebracht zu werden, und eine Gegenform (6, 6') umfasst, welche eine aus einem Antihaftmaterial bestehende Seite umfasst, die dazu bestimmt ist, mit dem formbaren Klebematerial (3) in Kontakt zu kommen.

3. Verfahren nach Anspruch 2, **dadurch gekennzeichnet, dass** die vorgenannte Walz-Form-Station kontinuierlich beschickt wird und eine erste Walze (5), welche eine Vielzahl von Eindrücken jeweils mit der allgemeinen Form des herzustellenden Verbands aufweist, sowie eine zweite Walze (6) umfasst, deren Außenfläche im Wesentlichen eben und glatt ist und aus einem Antihaftmaterial besteht.

4. Herstellungsverfahren nach Anspruch 2, **dadurch gekennzeichnet, dass** die vorgenannte Walz-Form-Station diskontinuierlich beschickt wird und zwei aufeinander zu bewegbare Platten umfasst, die erste Platte (5'), die eine Anordnung von Eindrücken umfasst, die jeweils die allgemeine Form des zu erhaltenden Verbands aufweisen, und eine zweite Platte (6'), deren Oberfläche aus einem Antihaftmaterial besteht.

5. Verfahren nach einem der vorhergehenden Ansprüche, **dadurch gekennzeichnet, dass** es einen Schritt vor dem vorgenannten Schritt a) umfasst, der darin besteht, die Walz-Form-Station mit einer Anordnung bestehend aus einer Trägerfolie (1), die auf einer ihrer Seiten eine Schicht aus einem formbaren Klebematerial (3) trägt, zu beschicken, wobei die Anordnung durch Beschichten, Extrudieren oder durch Abscheiden erhalten wird.

6. Verfahren nach einem der vorhergehenden Ansprüche, **dadurch gekennzeichnet, dass** der (die) Wirkstoff(e) in fester Form, beispielsweise in Form eines Pulvers, oder in flüssiger Form, beispielsweise in Form einer Lösung oder einer Suspension, oder aber in halbflüssiger Form, wie zum Beispiel in Form einer Paste oder eines Gels, aufgebracht wird (werden).

7. Verfahren nach Anspruch 6, **dadurch gekennzeichnet, dass** der (die) Wirkstoff(e) mittels eines Pulverspenders (12), vorzugsweise im Bereich der Teile des formbaren Klebematerials, welche dazu bestimmt sind, die mittleren Teile der Verbände zu bilden, in Pulverform aufgebracht wird (werden).

8. Verfahren nach Anspruch 7, **dadurch gekennzeichnet, dass** der (die) Wirkstoff(e) mittels einer Pistole oder einer Düse, vorzugsweise im Bereich der Teile des formbaren Klebematerials, welche dazu bestimmt sind, die mittleren Teile der Verbände zu bilden, in Form einer Paste oder eines Gels aufgebracht wird (werden).

## Claims

1. A method for manufacturing wound dressings, preferably with thinned edges, **characterized in that** it comprises the steps consisting in:
a) forming a first laminated element by joining together a support film (1) and a layer of an adhesive malleable material (3) intended to be brought into contact with the wound and containing:
- one or more elastomers selected from the poly(styrene-olefin-styrene) block copolymers,
- one or more compounds selected from so-called "tackifying products", antioxidants, liquid plasticizers and cohesion-improving agents selected from butylic rubbers or polyisobutylenes of high molecular weight,
- hydrocolloids;
b) shaping said laminated element to obtain an intermediate product (7) comprising a plurality of zones each forming a dressing preform;
said steps a) and b) are carried out simultaneously in a laminating-molding station;
c) treating the intermediate product (7) thus obtained to deposit one or more active substances on the free surface of said adhesive malleable material;
d) forming a second laminated element (10) by joining together said intermediate product (7) thus treated and a protective film (8);
e) cutting said second laminated element (10) thereby to form a plurality of dressings.

2. The manufacturing method as claimed in claim 1, **characterized in that** said laminating-molding station comprises a mold (5, 5') intended to be brought into contact with the support film (1) and an oddside mold (6, 6') comprising one side consisting of an anti-adhesive material intended to be in contact with the adhesive malleable material (3).

3. The method as claimed in claim 2, **characterized in that** the abovementioned laminating-molding station is supplied continuously and comprises a first roll (5) having a plurality of cavities each having the general shape of the dressing to be prepared and a second roll (6) whereof the outer surface is substantially plane and smooth and consists of an anti-adhesive material.

4. The manufacturing method as claimed in claim 2, **characterized in that** the abovementioned laminating-molding station is supplied in batches and comprises two plates movable toward one another, the first plate (5') comprising a set of cavities each having the general shape of the dressing to be obtained, and a second plate (6') whereof the surface consists of an anti-adhesive material.

5. The method as claimed in any one of the preceding claims, **characterized in that** it comprises a step prior to the abovementioned step a) consisting in supplying the laminating-molding station with a combination formed of a support film (1) bearing on one of its sides a layer of an adhesive malleable material (3), said combination being obtained by coating, extrusion or by deposition.

6. The method as claimed in any one of the preceding claims, **characterized in that** said active substance(s) is(are) deposited in solid form, for example in powder form, or in liquid form, for example in solution or suspension form, or even in semi-liquid form such as, for example, in paste or gel form.

7. The method as claimed in claim 6, **characterized in that** said active substance(s) is(are) deposited in powder form using a powder distributor (12), preferably at the parts of the adhesive malleable material intended to constitute the central parts of the dressings.

8. The method as claimed in claim 7, **characterized in that** said active substance(s) is(are) deposited in paste or gel form, using a gun or a nozzle, preferably at the parts of the adhesive malleable material intended to constitute the central parts of the dressings.
